# EUROPEAN PATENT SPECIFICATION

(11) **EP 3 873 562 B1**
(45) Date of publication and mention of the grant of the patent: **13.08.2025**
(21) Application number: 19861483.6
(22) Date of filing: 17.09.2019
(51) Int. Cl.: A61M 5/00, A61M 5/19, A61M 5/20, A61M 5/50, A61M 5/24, A61M 5/315

(54) **MINIATURIZED WEARABLE MEDICATION ADMINISTRATION DEVICE**
MINIATURISIERTE, AM KÖRPER TRAGBARE MEDIKAMENTENVERABREICHUNGSVORRICHTUNG
DISPOSITIF PORTATIF D'ADMINISTRATION DE MÉDICAMENT MINIATURISÉ

(30) Priority: 17.09.2018 US 201862732442 P
(43) Date of publication of application: 08.09.2021
(73) Proprietor: Rx Bandz, Inc., Locust Valley, NY 11560 (US)
(72) Inventor: WALSH, Jessica, Upper Brookville, NY 11771 (US); DINESEN, John, Austin, TX 78732 (US); HARHEN, Stephen, Austin, TX 78732 (US)
(74) Representative: Maidment, Marc
(86) International application number: PCT/US2019/051589
(87) International publication number: WO 2020/061100

(56) References cited:
- WO-A1-2016/009165
- US-A- 4 755 169
- US-A- 5 024 656
- US-A1- 2008 132 840
- US-A1- 2010 094 254
- US-A1- 2011 306 929
- US-A1- 2016 158 462
- US-A1- 2017 021 104
- US-A1- 2017 232 207
- US-A1- 2017 246 393
- US-A1- 2017 361 016
- US-A1- 2018 110 925
- US-A1- 2018 193 557
- US-A1- 2018 304 017
- US-B2- 9 474 862
- V-GO SIMPLE INSULIN PATCH: "How V-Go® Works", 7 December 2017 (2017-12-07), XP054982078, Retrieved from the Internet <URL:https://www.youtube.com/watch?v=OupIc7ts-mw>

## Description

### Field of the Invention

The field of invention relates to a wearable device for administration of medication to a subject in need thereof. In particular, the field of invention relates to a miniaturized needle-based autoinjector.

### Background of the Invention

Devices have been used to administer medications under emergency conditions, such as, for example, administering epinephrine to counteract the effects of a severe allergic reaction (e.g., anaphylaxis) or naloxone for opioid overdose. Devices have also been described for use in administering medications to treat disease, such as, for example, anti-arrhythmic medications and selective thrombolytic agents during a heart attack. Autoinjectors offer an alternative to manually operated syringes for administering therapeutic agents into subjects in need thereof, or allowing subjects in need thereof to self-administer medications. Frequently, autoinjectors administer medication to the subject via injection.

Current autoinjectors are frequently cumbersome and may not be readily accessible, or easily carried by the user. In particular, due to the bulk of the current autoinjectors, the devices tend to be carried in the subject's bag, or back pack, or purse, or car, not on the subject's person. In addition, the currently available autoinjectors have a number of problems in form, function, and appeal. These problems can contribute to incorrect use, misuse, not carrying the unit as prescribed (non-compliance), and can result in an adverse outcome including death. By way of example, anaphylaxis is a severe medical emergency that if not treated quickly and appropriately can be fatal. It occurs unexpectedly and may progress rapidly in patients of all ages, but most often in the young and otherwise healthy. One common cause of anaphylaxis is food allergy, especially to peanuts, which is increasing in prevalence. Other causes exist, such as, for example, other food allergens, adverse reactions to medications, or adverse reactions to insect bites or stings. Rapid diagnosis is essential and immediate injection of intramuscular epinephrine is the treatment of choice, the response to which is often dramatic and potentially life-saving. The early injection of epinephrine is the most important factor in anaphylaxis outcome. People who survive near fatal anaphylactic reactions receive intramuscular injections promptly while those who die do not.

Known medication administration devices are disclosed in US 2008/132840 A1 and US 2011/306929 A1.

### Brief Description of the Figures

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.
Figure 1 shows an exploded perspective view of an exemplary miniaturized wearable medication administration device.
Figure 2 shows an assembled perspective view of the device of Figure 1.
Figure 3 shows a perspective view of a quick release mount that may interface with the device of Figure 1.
Figure 4 shows a perspective view of the quick release mount of Figure 3 and a receiving portion of the device of Figure 1.
Figure 5 shows a cross-sectional view of the device of Figure 1 and a garment incorporating the quick release mount of Figure 3.
Figure 6 shows a partially transparent perspective view of the device of Figure 1 at a time of activation.
Figure 7 shows a partially transparent perspective view of the device of Figure 1 at a first intermediate stage of deployment.
Figure 8 shows a partially transparent perspective view of the device of Figure 1 at a second intermediate stage of deployment.
Figure 9 shows a partially transparent perspective view of the device of Figure 1 at a third intermediate stage of deployment.
Figure 10 shows a partially transparent perspective view of the device of Figure 1 after deployment has been completed.
Figure 11 shows an exploded perspective view of an exemplary miniaturized wearable medication administration device.
Figure 12 shows a top perspective view of the exemplary miniaturized wearable medication administration device of Figure 11 with a slide plate removed therefrom.
Figure 13 shows a bottom perspective view of the exemplary miniaturized wearable medication administration device of Figure 11 with a slide plate attached thereto.

### Summary of the Invention

The invention is defined by independent claim 1. In an embodiment, a device includes a housing having a first end, a second end, a first cylinder, a second cylinder, a channel connecting the first cylinder to the second cylinder proximate the second end, a piston slidably positioned within the first cylinder, a spring configured to force the piston toward the second end of the housing, an activation trigger positioned on the first end of the housing and configured to retain the piston proximate the first end of the housing prior to activation and to allow the piston to move along the first cylinder upon activation, a penetrable septum positioned on the first end of the housing adjacent to the activation trigger, a cartridge having a first end and a second end, the cartridge containing a therapeutic agent, the cartridge being positioned within the second cylinder such that the first end of the cartridge faces the first end of the housing and the second end of the cartridge faces the second end of the housing, the cartridge having a plunger at the second end thereof, a cartridge sealing ring positioned adjacent the second end of the cartridge, a needle hub positioned adjacent the first end of the cartridge and within the second cylinder such that the cartridge is positioned within the needle hub a dual-ended needle positioned within the needle hub, a retraction spring positioned within the second cylinder and configured to force the needle hub toward the second end of the cylinder, and a fluid positioned within the first cylinder, the channel, and the second cylinder, whereby, when the piston travels along the first cylinder toward the second end of the housing, the piston drives some of the fluid through the channel into the second cylinder, thereby driving the cartridge sealing ring, the cylinder, the needle hub, and the needle toward the first end of the housing, thereby causing the needle to penetrate the septum and subsequently to penetrate the cartridge, wherein continued travel of the fluid causes the plunger to move within the cartridge and extrude at least some of the therapeutic agent through the needle, wherein the first cylinder includes a relief bore positioned proximate the second end of the housing, the relief bore configured such that, when a head of the piston is positioned between the relief bore and the second end of the housing, the fluid is permitted to flow into a portion of the first cylinder between the head of the piston and the first end of the housing, thereby removing pressure from the cartridge sealing ring, and wherein the retraction spring is configured to force the needle hub toward the second end of the housing when pressure is removed from the cartridge sealing ring, thereby causing corresponding motion of the needle toward the second end of the housing and retracting the needle so as to be positioned entirely within the housing.

In an embodiment, a device includes a housing having a first end, a second end, a first cylinder, a second cylinder parallel to the first cylinder, and a channel connecting the first cylinder to the second cylinder proximate to the second end; a piston slidably positioned within the first cylinder; a deployment spring configured to force the piston toward the second end of the housing; an activation trigger positioned proximate to the first end of the housing, wherein the activation trigger is configured to retain the piston proximate the first end of the housing prior to actuation of the activation trigger, and wherein the activation trigger is configured to release the piston and to allow the piston to move along the first cylinder upon actuation of the activation trigger; a penetrable septum positioned at the first end of the housing proximate to the activation trigger, wherein the penetrable septum separates an interior of the housing from an external environment surrounding the housing; a cartridge having a first end and a second end, wherein the cartridge contains at least one therapeutic agent, wherein the cartridge is positioned within the second cylinder such that the first end of the cartridge faces the first end of the housing and the second end of the cartridge faces the second end of the housing, and wherein the cartridge has a plunger at the second end thereof; a needle hub positioned adjacent to the first end of the cartridge and within the second cylinder, wherein the cartridge is slidably positioned within the needle hub; a dual-ended needle positioned within the needle hub; and a fluid positioned within the first cylinder, the channel, and the second cylinder, wherein the piston is configured such that, when the activation trigger is actuated, the activation trigger releases the piston and the deployment spring causes the piston to travel along the first cylinder toward the second end of the housing, thereby driving at least some of the fluid through the channel into the second cylinder, thereby driving the cartridge, the needle hub, and the needle toward the first end of the housing, and thereby causing the needle to penetrate the septum and subsequently to penetrate the cartridge; and wherein the plunger is configured such that continued travel of the fluid causes the plunger to move within the cartridge and force at least some of the at least one therapeutic agent to extrude through the needle.

In an embodiment, the device also includes a retraction spring positioned within the second cylinder, wherein the retraction spring is configured to force the needle hub toward the second end of the cylinder. In an embodiment, the piston includes a piston head, the first cylinder includes a relief bore positioned proximate to the second end of the housing, the relief bore is configured such that, when the piston head is positioned between the relief bore and the second end of the housing, the fluid is permitted to flow into a portion of the first cylinder between the head of the piston and the first end of the housing, thereby removing pressure from the cartridge sealing ring, and the retraction spring is configured to force the needle hub toward the second end of the housing when pressure is removed from the cartridge sealing ring, thereby causing corresponding motion of the needle toward the second end of the housing and retraction of the needle so as to be positioned entirely within the housing. In an embodiment, a position of the relief bore is configured so as to control an amount of the at least one therapeutic agent to be extruded through the needle. In an embodiment, the deployment spring and the retraction spring are configured such that an elapsed time between an actuation of the activation trigger and a subsequent retraction of the needle is less than 2.0 seconds.

In an embodiment, the fluid is one of a hydraulic fluid or a pneumatic fluid.

In an embodiment, a length of the device is between 10 millimeters and 67.5 millimeters. In an embodiment, a height of the device is between 5 millimeters and 16 millimeters. In an embodiment, a width of the device is between 5 millimeters and 27 millimeters.

In an embodiment, the device also includes a clip configured to removably attach the device to one of a person or an item. In an embodiment, the clip includes at least one of a slide-on clip or a pinch clip.

In an embodiment, the device also includes a cartridge sealing ring positioned adjacent to the second end of the cartridge, wherein the fluid is configured to drive the cartridge sealing ring toward the first end of the housing, thereby driving the cartridge toward the first end of the housing.

In an embodiment, the housing is configured to reflect infrared radiation.

In an embodiment, the housing includes a semi-permeable barrier overlaying the penetrable septum. In an embodiment, the semi-permeable barrier comprises flash-spun high-density polyethylene. In an embodiment, the housing is fluid-tight.

In an embodiment, the at least one therapeutic agent is a plurality of therapeutic agents.

In an embodiment, the device also includes at least one sensor.

### Detailed Description of the Invention

Among those benefits and improvements that have been disclosed, other objects and advantages of this invention will become apparent from the following description taken in conjunction with the accompanying figures. Detailed embodiments of the present invention are disclosed herein; however, it is to be understood that the disclosed embodiments are merely illustrative of the invention that may be embodied in various forms. In addition, each of the examples given in connection with the various embodiments of the invention which are intended to be illustrative, and not restrictive.
Throughout the specification and claims, the following terms take the meanings explicitly associated herein, unless the context clearly dictates otherwise. The phrases "in one embodiment," "in an embodiment," and "in some embodiments" as used herein do not necessarily refer to the same embodiment(s), though it may. Furthermore, the phrases "in another embodiment" and "in some other embodiments" as used herein do not necessarily refer to a different embodiment, although it may. Thus, as described below, various embodiments of the invention may be readily combined.
As used herein, the term "based on" is not exclusive and allows for being based on additional factors not described, unless the context clearly dictates otherwise. In addition, throughout the specification, the meaning of "a," "an," and "the" include plural references. The meaning of "in" includes "in" and "on."
Unless otherwise defined, all terms (including technical and scientific terms used herein have the same meaning as commonly understood by one having ordinary skill in the art to which this invention belongs. It will further be understood that terms, such as those defined, in commonly used dictionaries, should be interpreted as having a meaning that is consistent with their meaning in the context of the relevant art and the present disclosure and will not be interpreted in an idealized or overly formal sense unless expressly defined.
The present disclosure describes an exemplary embodiment of a miniaturized wearable injection device. It will be apparent to those of skill in the art that the general principles embodied in the exemplary device may also be embodied in other devices. In some embodiments, an exemplary miniaturized wearable injection device is suitable for use as an autoinjector, i.e., a device that is self-administered by a patient who is experiencing an emergency health condition.

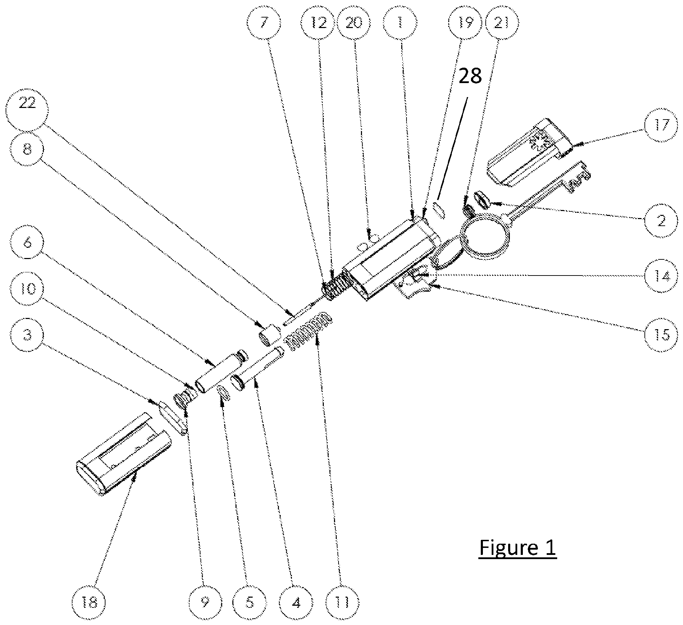

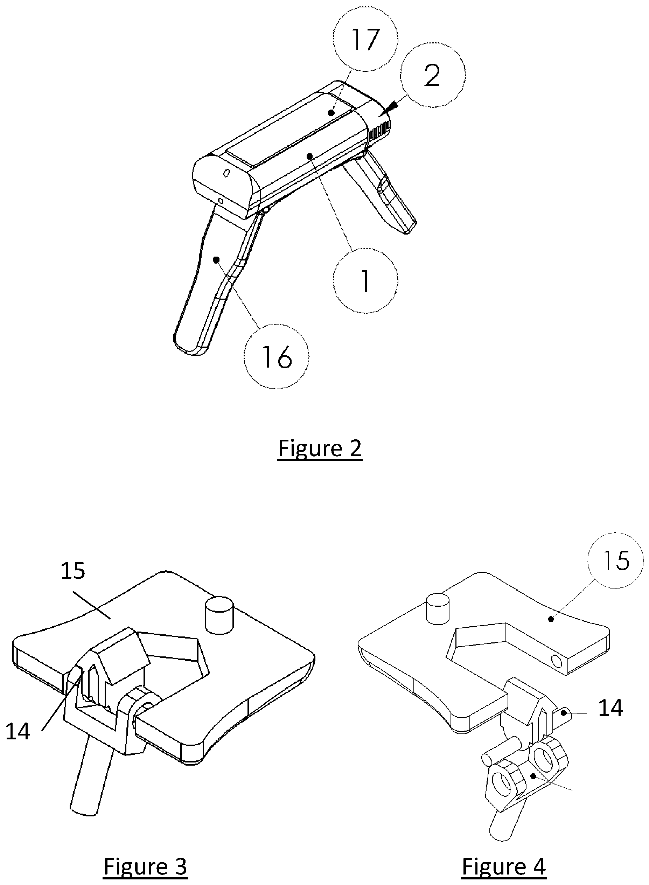

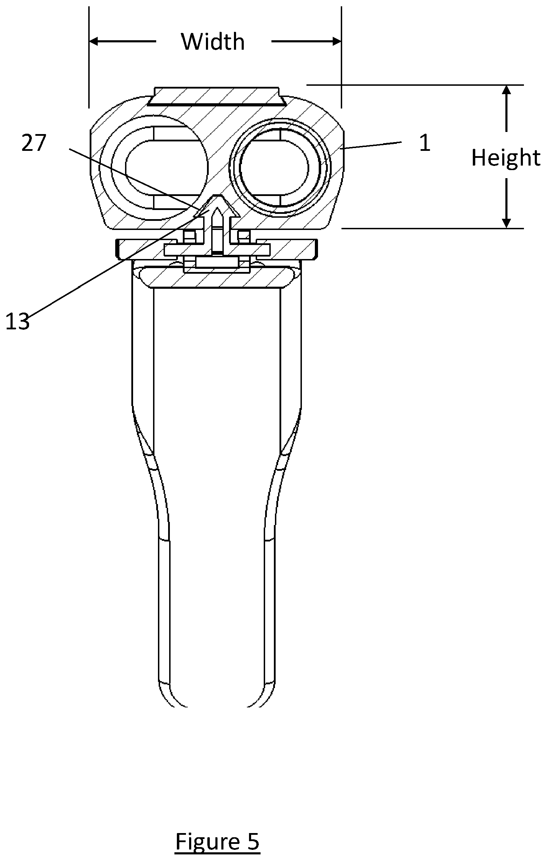

Figure 1 shows an exploded perspective view of the elements of an exemplary autoinjector 100 (hereinafter the "device 100"). In some embodiments, the device 100 includes a housing 1. In some embodiments, the housing 1 is configured to be removably fastened to a quick release, as shown in Figure 3. In some embodiments, the quick release is integrated into an accessory, such as a watch band 16. It will be apparent to those of skill in the art that the quick release 13 can be integrated into another item to which a user may wish to affix the device 100 (e.g., another accessory, a bag, a key chain, a backpack, a mobile phone case, a bracelet, a key ring, a bag, a lanyard, a magnet, a mobile phone, a pin, a belt, a carabiner, a backpack clip, a zipper pull, a wallet, a belt clip, etc.). In some embodiments, the quick release includes a housing clip 14 (see Figure 3) and the housing 1 includes a recess 27 (see Figure 5) that is sized and shaped to removably receive the housing clip 14. In some embodiments, the quick release includes a pinch clip 15 that is configured such that, when pinched, the pinching force is conveyed to the housing clip 14, thereby allowing the housing clip 14 to be removed from the housing 1 (and, consequently, allowing the device 100 to be removed from the item to which it was attached by the quick release). In some embodiments, the quick release 13 includes a slide-on clip.

In some embodiments, the device 100 includes a cartridge 6 storing a therapeutic agent (e.g., a pharmaceutical). In some embodiments, the cartridge 6 contains 1 mL of the therapeutic agent. In some embodiments, the cartridge 6 contains a mixture including one or more therapeutic agents. In some embodiments, the cartridge has an outside diameter of 9.85 mm. In some embodiments, the cartridge has a length of 35 mm. In some embodiments, the cartridge has a length of 51 mm. In some embodiments, the cartridge has a length of between 46 mm and 56 mm. Examples of therapeutic agents suitable for use in the device 100 include glucagon, insulin, adrenaline, epinephrine, anti-venom, atropine, antibody formulations, antidotes to chemical agents, and the like. In some embodiments, the medication suitable for use in the device of the present invention is at least one medication selected from the group of medications identified by tradenames consisting of Acthar, Actimmune, Apokyn, AquaMephyton, Aranesp, Arixtra, Avonex, Betaseron, Bravelle, Butorphanol, Byetta, Calcijex, Calcitonin, Caverject, Cetrotide, Chorionic Gonadotropin, Cimzia, Copaxone, Copegus, DDAVP, D.H.E-45, Delatestryl, Delestrogen, Depo-Estradiol, Depo-Provera 150, Depo-SubQ Provera 104, Depo-Testosterone, Desmopressin, Dihydroergotamine, Edex, Eligard, Enbrel, Epipen, Epogen, Exjade, Faslodex, Fertinex, Follistim, Forteo, Fragmin, Fuzeon, Ganirelix acetate, Genotropin, Gleevec, Glucagon, Gonal, Heparin, Humatrope, Humira, Imitrex, Increlex, Infergen, Innohep, Insulin, Intron A, iPlex, Ketorolac, Kestrone, Kineret, Kuvan, Leukine, Leuprolide Acetate, Lovenox, Lupron, Luveris, Medroxyprogesterone, Menopur, Methotrexate, Miacalcin, Muse, Neumega, Neulasta, Neupogen, Nexavar, Norditropin, Novarel, Nutropin, NuvaRing, Omnitrope, Orfadin, Ovidrel, Pegasys, Peg-Intron, Pregnyl, Procrit, Profasi, Progesterone, Pulmozyme, Raptiva, Rebetol, Rebif, Repronex, Revlimid, Ribasphere, Ribavirin, Saizen, Sandostatin, Sensipar, Serostim, Somatuline, Sprycel, Somavert, Stadol, Sumatriptan, Supprelin, Sutent, Symlin, Tarceva, Testosterone, Temodar, Tev-Tropin, Thalomid, Tobi, Tykerb, Vitamin B12, Vitamin D, Vitamin K, Xeloda, Zemplar, and Zorbtive. In some embodiments, the device 100 is capable of delivering highly viscous therapeutic agents (e.g., having a viscosity greater than that of water, having a viscosity greater than 30 centipoise, having a viscosity between 30 centipoise and 50 centipoise, etc.), such as biologicals or monoclonal antibodies. It will be apparent to those of skill in the art that the therapeutic agents listed above are only exemplary and that the device 100 is equally suitable for use in connection with practically any other injectable therapeutic agent not specifically listed herein.

In some embodiments, the device 100 is environmentally robust to allow for the device 100 to be worn in a variety of environments while maintaining the stability of the therapeutic agent in the cartridge 6. In some embodiments, the housing 1 is made from a polycarbonate plastic to shield the cartridge 6 from stress and ultraviolet light. In some embodiments, a septum 19 seals a first end of the housing 1. In some embodiments, the housing 1 and the septum 19 are sealed so as to prevent ingress of dust, water, and other contaminants. In some embodiments, the housing 1 provides visibility to the cartridge 6 (e.g., so that the user can read a label). In some embodiments, the housing 1 is at least partially transparent. In some embodiments, the housing 1 has a viewing window providing visibility of the cartridge 6. In some embodiments, a removable cap 17 is provided to cover the viewing window.

In some embodiments, the housing 1 is configured to thermally insulate the therapeutic agent contained within the cartridge 6 from excursions into temperatures that would otherwise cause rapid degradation of the therapeutic agent (e.g., exposure to high levels of heat). In some embodiments, the housing 1 provides for the maintenance of a vacuum on the interior of the housing 1. In some embodiments, the housing 1 includes material insulation. In some embodiments, the housing 1 is formed from a plastic that includes one or more infrared ("IR") reflective additives. In some embodiments, the IR reflective additives include one or more of titanium oxide, bismuth vanadate yellow, chrome antimony titanium buff rutile, chrome iron brown hematite, chrome oxide green, chromium green-black hematite, cobalt aluminate blue spinel, cobalt chromite blue-green spinel, cobalt chromite green spinel, cobalt titanate green spinel, iron manganese black oxide, iron titanium brown spinel, manganese antimony titanium buff rutile, nickel antimony titanium yellow rutile, red iron oxide, ultramarine blue, ultramarine violet, zinc ferrite brown spinel, or another suitable additive. In some embodiments, the one or more IR reflective additives are selected to provide a desired color for the housing 1. In some embodiments, the housing is shielded from stress, heat and radiation via a reflective insulative coating 18.

In some embodiments, the removable cap 17 provides infrared reflection. In some embodiments, the removable cap 17 is configured to slide off of the housing 1 to reveal the cartridge 6, an indication 20 of the drug temperature, and labeling on the top of the housing 1. In some embodiments, the removable cap 17 is configured to provide the drug with environmental protection and discrete labeling. In some embodiments, the removable cap 17 provides a primary and reversible safety for an activation button 2 that is disposed on the housing 1. In some embodiments, the removable cap 17 is configured to be capable of removal via a thumb push with finger grips as seen in Figure 2 on the head of the removable cap 17 to allow for a two-finger pinch removal. In some embodiments, the housing 1 and the removable cap 17 are configured such that instructions and labelling are concealed by the removable cap 17 until time of use, when the removable cap 17 is removed. In some embodiments, the device 100 provides voice instructions for use. In some embodiments, the housing 1 includes an integrated information display that is concealed by the removable cap 17. In some embodiments, the device 100 includes a compartment to house pills. In some embodiments, the device 100 includes integrated sensors or other integrated electronics. In some embodiments, the device 100 includes one or more integrated sensors that are configured to sense one or more parameters relating to the device 100 and/or relating to a patient.

In some embodiments, the device 100 includes an activation trigger 2 extending from the housing 1 adjacent to the septum 19. In some embodiments, to activate the device 100, a user removes the removable cap 17 from the housing 1 and presses the housing 1 against an injection site (e.g., against skin or against clothing overlaying the injection site) so as to position the septum 19 at the injection site and press the activation trigger 2 against the skin, thereby triggering administration of the therapeutic agent as will be described hereinafter. In some embodiments, a back plate 3 seals the housing 1 at an end opposite the activation trigger 2. In some embodiments, the cap 17 and the activation trigger 2 provide the device 100 with two safety levels before injection: the cap 17 acts as a primary and reversible safety, while the activation trigger 2 acts as a non-reversible safety. In some embodiments, the cap 17 includes ridged posts that prevent activation of the activation trigger 2 while the cap is in place. In some embodiments, the device 100 includes one or more additional safety mechanisms. In some embodiments, the activation trigger 2 is configured to remain depressed after activation, thereby providing a visual indication that the device 100 has been activated.

In some embodiments, when the activation trigger 2 is pressed, a stored energy system is released. In some embodiments, when the activation trigger 2 is pressed, a deployment spring 11 drives a piston 4 having an O-ring 5. In some embodiments, rather than the deployment spring 11, the device 100 includes a stored energy system that incorporates the release of a pressurized fluid to drive the piston 4 upon activation of the device 100, that incorporates the activation of an electric motor to drive the piston 4 upon activation of the device 100, or that drives the piston 4 in some other manner upon activation of the device 100. In some embodiments, the piston 4 is locked into place by a split lock that engages a portion of the housing 1, and the activation trigger 2 has a conical shape that releases the split lock and allows the piston 4 to move along the housing 1. In some embodiments, the device 100 includes a button spring 21 positioned so as to resist activation of the activation trigger 2, thereby preventing inadvertent activation of the device 100. In some embodiments, once the piston 4 has been unlocked, the deployment spring 11 drives the piston 4 along a first cylinder 23 within the housing 1 in a direction away from the activation trigger 2. In some embodiments, the piston 4 correspondingly drives a fluid (e.g., a hydraulic fluid, a pneumatic fluid, etc.) within the first cylinder 23, through a channel 24 at the end of the housing 1 opposite the activation trigger 2, and along a second cylinder 25, thereby driving a cartridge sealing ring 9, the cartridge 6, and a needle hub 8 toward the septum 19 in a manner so as to provide correct injection depth and volume via a double-ended needle 7 and a rubber plunger 10 as will be described hereinafter. In some embodiments, the fluid is a hydraulic fluid. In some embodiments, the hydraulic fluid includes either glycol, alcohol, or calcium chloride. In some embodiments, the fluid is a pneumatic fluid. In some embodiments, rather than using the deployment spring 11, a stored energy system includes a compressed gas utilizing a valve to control step progress. In some embodiments, the needle hub 8 is crushable (i.e., compressible).

In some embodiments, the needle hub 8 is configured to stabilize the cartridge 6 and align the cartridge 6 and the needle 7 for penetration of the cartridge 6 by the needle 7. In some embodiments, the needle 7 is fixed to the needle hub 8. In some embodiments, the needle 7 is glued to the needle hub 8. In some embodiments, the needle 7 is overmolded into the needle hub 8. In some embodiments, the needle 7 is positioned within a needle sheath 22 prior to deployment of the device 100. In some embodiments, the needle sheath 22 maintains sterility of the needle 7 during assembly of the device 100 and is molded with the needle 7 into the needle hub 8 during assembly of the device 100. In some embodiments, the needle sheath 22 is crushable.

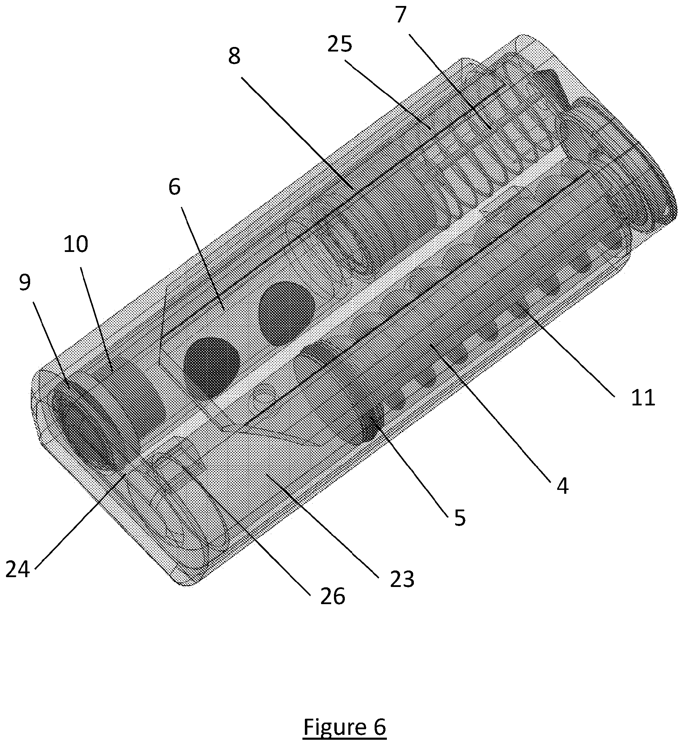

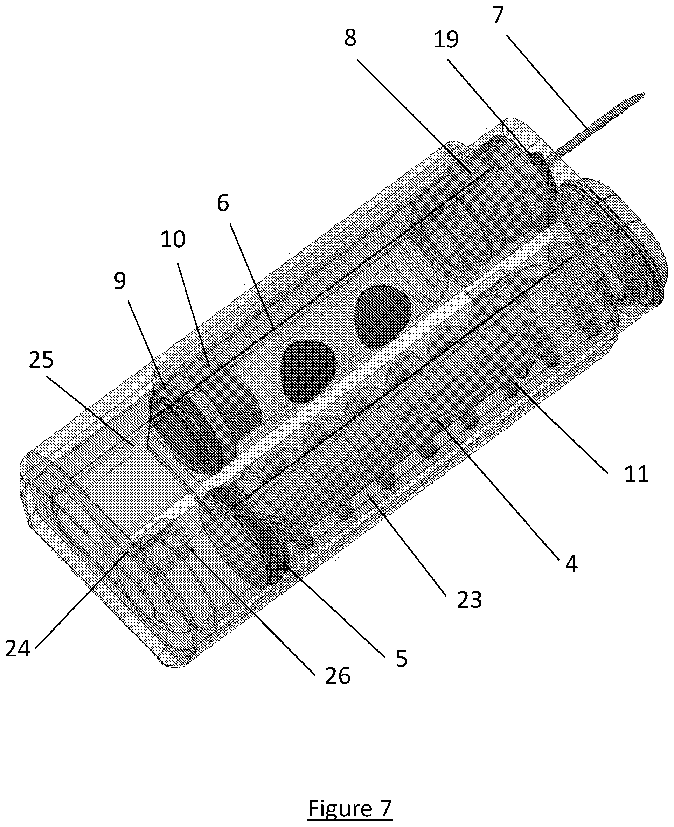

In some embodiments, the piston 4 passes through certain discrete positions as it travels along the first cylinder 23. Figure 6 shows the device 100 with the activation trigger 2 depressed so as to unlock the piston 4, but prior to the piston 4 traveling along the first cylinder 23. In some embodiments, as the piston 4 travels along the first cylinder 23, it first passes through a first intermediate position, as shown in Figure 7. In some embodiments, as the piston 4 travels along the first cylinder 23 from the position shown in Figure 6 to the position shown in Figure 7, the piston 4 drives the fluid along the first cylinder 23, through the channel 24, and through the second cylinder 25. The movement of the fluid drives the cartridge sealing ring 9, the cartridge 6, the needle hub 8, and the needle 7 toward the septum 19, thereby forcing the needle 7 through the septum 19 and exposing the needle 7. In some embodiments, a sufficient amount of the needle is exposed so as to allow for sufficient injection depth for intramuscular injection. In some embodiments, the amount is at least 10 millimeters. In some embodiments, the amount is at least 12.9 millimeters. In some embodiments, the amount is 12.9 millimeters. In some embodiments, the position shown in Figure 7 is defined by the needle hub 8 having impacted the housing 1 adjacent to the septum 19.

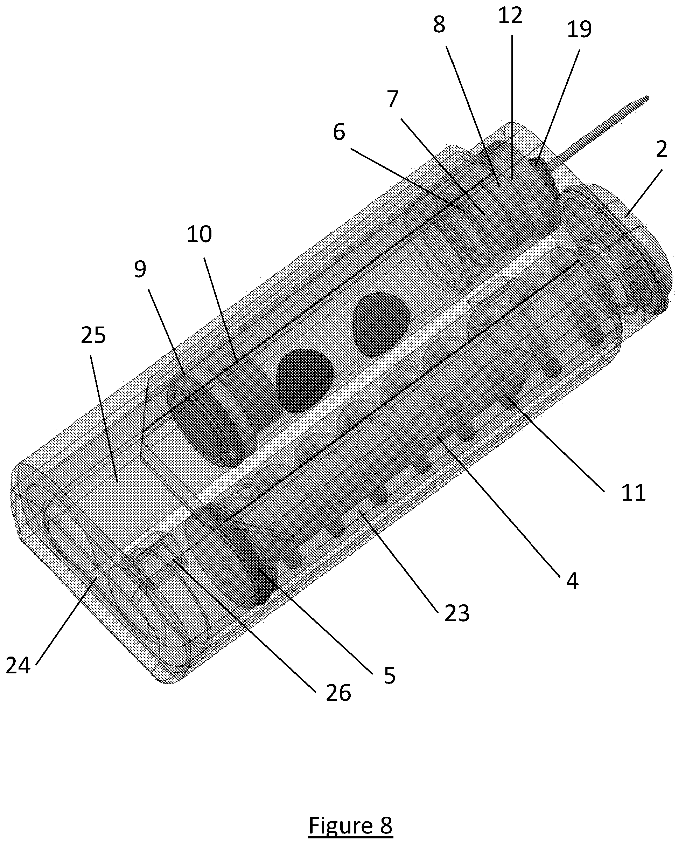

In some embodiments, the piston 4 continues to be forced along the first cylinder 23 by the deployment spring 11, causing motion from the position shown in Figure 7 to the position shown in Figure 8. In some embodiments, as the piston 4 travels along the first cylinder 23 from the position shown in Figure 7 to the position shown in Figure 8, the piston 4 continues to drive the fluid along the first cylinder 23, through the channel 24, and through the second cylinder 25. In some embodiments, the fluid drives the cartridge sealing ring 9 and the cartridge 6 toward the septum 19, thereby advancing the cartridge 8 within the needle hub 8 and driving the cartridge 6 toward the needle 7, and thereby causing the needle 7 to penetrate the cartridge 6. In some embodiments, the position shown in Figure 8 is defined by the maximum possible travel of the cartridge 8 within the needle hub 8 under the force provided by the deployment spring 11 and conveyed via the fluid.

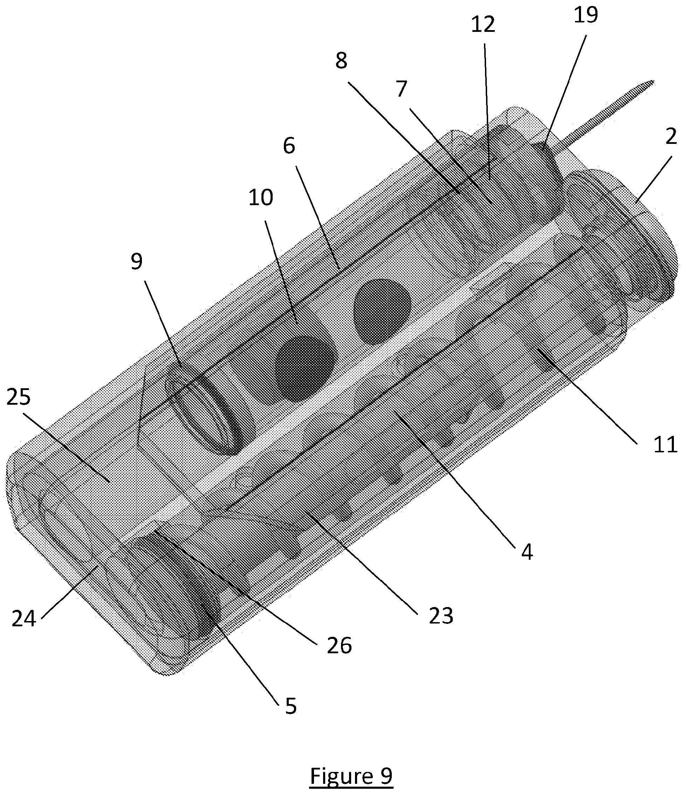

In some embodiments, the piston 4 continues to be forced along the first cylinder 23 by the deployment spring 11, causing motion from the position shown in Figure 8 to the position shown in Figure 9. In some embodiments, as the piston 4 travels along the first cylinder 23 from the position shown in Figure 8 to the position shown in Figure 9, the piston 4 continues to drive the fluid along the first cylinder 23, through the channel 24, and through the second cylinder 25. In some embodiments, the fluid drives the cartridge sealing ring 9 toward the septum 19. In some embodiments, further movement of the cartridge 6 toward the septum 19 is constrained by the available compression of the needle hub 8. Consequently, in some embodiments, the movement of the cartridge sealing ring 9 toward the septum 19 causes the cartridge sealing ring 9 to drive a plunger 10 of the cartridge 6 toward the septum 19, thereby extruding the therapeutic agent contained within the cartridge 6 through the needle 7.

In some embodiments, continued motion of the piston 4 along the first cylinder 23, as caused by the deployment spring 11, drives the head of the piston 4 to a point such that it passes a pressure relief bore 26 within the first cylinder 23. In some embodiments, the pressure relief bore 26 is a widening (e.g., increase in diameter) of the first cylinder 23 near the end of the housing 1 opposite the activation trigger 2, such that the entire perimeter of the head of the piston 4 is no longer securely engaged with the walls of the first cylinder 23 once the head of the piston 4 has passed the pressure relief bore 26. In some embodiments, the pressure relief bore 26 does not extend entirely about the perimeter of the first cylinder 23. In some embodiments, once the O-ring 5 of the piston 4 has passed the relief ring, motion of the piston 4 no longer drives the fluid, but, rather, the fluid is permitted to travel behind the head of the piston 4 and along the first cylinder 23 toward the activation trigger 2. In some embodiments, once the piston 4 no longer drives the fluid, the pressure on the plunger 10 described in the previous paragraph is relieved, and the therapeutic agent ceases to be extruded through the needle 7. In some embodiments, the piston 4 continues to drive the fluid through the channel 24, thereby driving the plunger 10 and extruding the therapeutic agent through the needle 7, until the piston 4 has passed the pressure relief bore 26. Consequently, in some embodiments, the location of the pressure relief bore 26 may be configured to control an amount of the therapeutic agent that is extruded through the needle 7. Figure 9 shows the piston 4 positioned past the pressure relief bore 26, allowing the fluid to flow into the first cylinder 23 behind the piston 4. Consequently, Figure 9 shows the plunger 10 at its maximum depth within the cartridge 6, i.e., an entire dose of the therapeutic agent has been administered. In some embodiments, the dosage amount is between 100 uM and 0.7 mL.

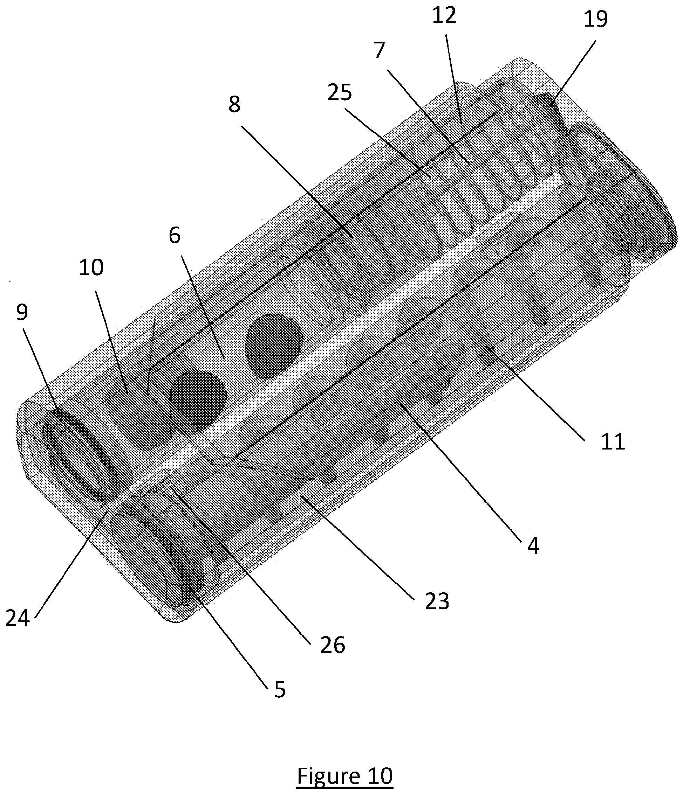

As noted above, in some embodiments, once the piston 4 has passed the pressure relief bore 26 and the fluid is permitted to flow behind the piston 4 into the first cylinder, the piston 4 will no longer drive the fluid through the channel 24 and along the second cylinder 25 to apply pressure to the cartridge sealing ring 9. Once pressure applied by the fluid has abated, a retraction spring 12 forces the needle hub 8, the cartridge 6, the cartridge sealing ring 9, and the needle 7 away from the septum 19 of the housing 1, thereby retracting the needle to a position that is entirely within the housing 1. In some embodiments, the retraction spring 12 is selected so as to be strong enough to provide an immediate or near-immediate retraction of the needle 7 once the pressure has abated, but not so strong as to prevent the cartridge 6 and associated elements from advancing toward the septum 19 while the pressure is being applied by the deployment spring 11 and the piston 4. Figure 10 shows the device 100 after the retraction spring 12 has retracted the cartridge 6 and other elements, thereby retracting the needle 7 within the housing 1.

In some embodiments, the deployment spring 11 and the retraction spring 12 are configured (e.g., selected to have an appropriate spring coefficient) such that the entire process of activation, deployment, administration of the therapeutic agent, and retraction is performed within a predefined period of elapsed time. In some embodiments, the predefined period of elapsed time is selected in order to be a clinically acceptable time period. In some embodiments, the period of elapsed time is less than 2.0 seconds. In some embodiments, the period of elapsed time is less than 1.5 seconds. In some embodiments, the period of elapsed time is less than 1.0 seconds. In some embodiments, the period of elapsed time is less than 0.5 seconds. In some embodiments, the period of elapsed time is between 0.2 seconds and 2.0 seconds. In some embodiments, the period of elapsed time is between 0.2 seconds and 1.5 seconds. In some embodiments, the period of elapsed time is between 0.2 seconds and 1.0 seconds. In some embodiments, the period of elapsed time is between 0.2 seconds and 0.5 seconds. In some embodiments, the period of elapsed time is between 0.5 seconds and 2.0 seconds. In some embodiments, the period of elapsed time is between 0.5 seconds and 1.5 seconds. In some embodiments, the period of elapsed time is between 0.5 seconds and 1.0 seconds. In some embodiments, the period of elapsed time is between 1.0 seconds and 2.0 seconds. In some embodiments, the period of elapsed time is between 1.0 seconds and 1.5 seconds. In some embodiments, the period of elapsed time is between 1.5 seconds and 2.0 seconds.

In some embodiments, the device 100 also includes a semi-permeable barrier 28 positioned adjacent to the septum 19. In some embodiments, the semi-permeable barrier 28 includes polyethylene. In some embodiments, the semi-permeable barrier 28 includes flash-spun high-density polyethylene ("HDPE"). In some embodiments, the semi-permeable barrier 28 includes flash-spun HDPE of the type commercialized by DuPont de Nemours, Inc. of Wilmington Delaware under the trade name TYVEK. In some embodiments, the semi-permeable barrier 28 can be penetrated by the needle 7 but is not permeable to water, thereby rendering the assembled device 100 waterproof. In some embodiments, the semi-permeable barrier 28 does not absorb moisture and the housing 1 of the device 100 is otherwise fluid-tight, thereby enabling the assembled device 100 to be sterilized using ethylene oxide sterilization or another liquid-based or gas-based sterilization technique.

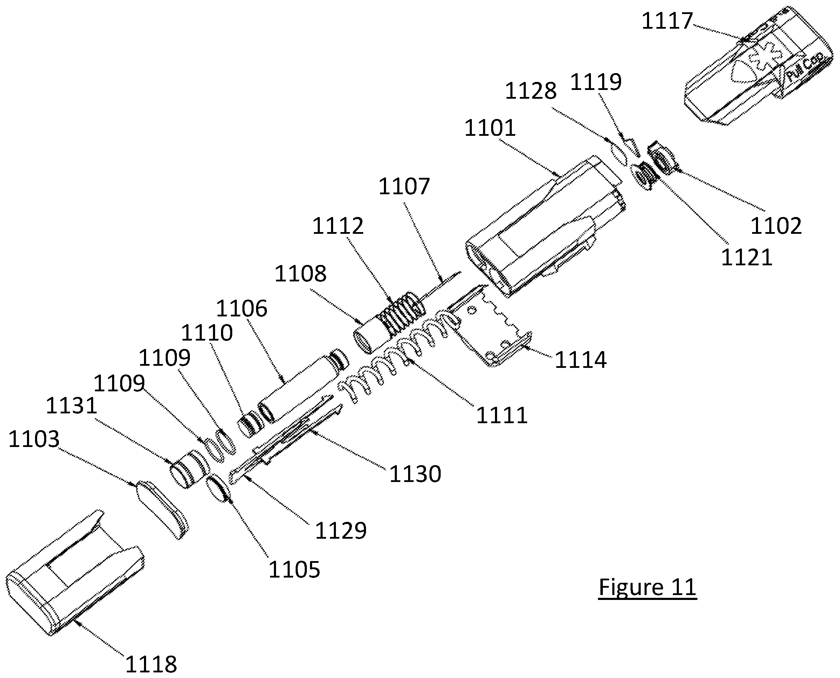

Figure 11 shows an exploded view of an exemplary device 1100. In some embodiments, the device 1100 is substantially similar to the device 100 discussed above other than as will be discussed in further detail hereinafter. In some embodiments, the device 1100 includes a housing 1101 having a first end and a second end. In some embodiments, the device 1100 includes an activation trigger 1102 positioned at the first end of the housing 1101. In some embodiments, the device 1100 includes a back plate 1103 sealing the housing 1101 at the second end of the housing 1101. In some embodiments, the device 1100 includes a two-piece piston including a first piston element 1129 and a second piston element 1130. In some embodiments, the piston includes an O-ring 1105. In some embodiments, the device 1100 includes a cartridge 1106. In some embodiments, the device 1100 includes a syringe 1107. In some embodiments, the device 1100 includes a needle hub 1108. In some embodiments, the device 1100 includes a cartridge sealing ring 1131. In some embodiments, the cartridge sealing ring 1131 carries one or more cartridge sealing O-rings 1109. In some embodiments, the cartridge sealing ring 1131 carries two of the O-rings 1109. In some embodiments, the device 1100 includes a plunger 1110. In some embodiments, the device 1100 includes a deployment spring 1111. In some embodiments, the device 1100 includes a retraction spring 1112. In some embodiments, the device 1100 includes a slide plate 1114. In some embodiments, the slide plate 1114 is configured to removably attach to the housing 1101 (e.g., by sliding onto and off of the housing 1101). In some embodiments, the device 1100 includes a removable cap 1117. In some embodiments, the device 1100 includes an insulative coating 1118. In some embodiments, the device 1100 includes a septum 1119. In some embodiments, the device 1100 includes a button spring 1121 positioned between the activation trigger 1102 and the two-piece piston and configured to facilitate deployment of the two-piece piston. In some embodiments, the device 1100 includes a semi-permeable barrier 1128.

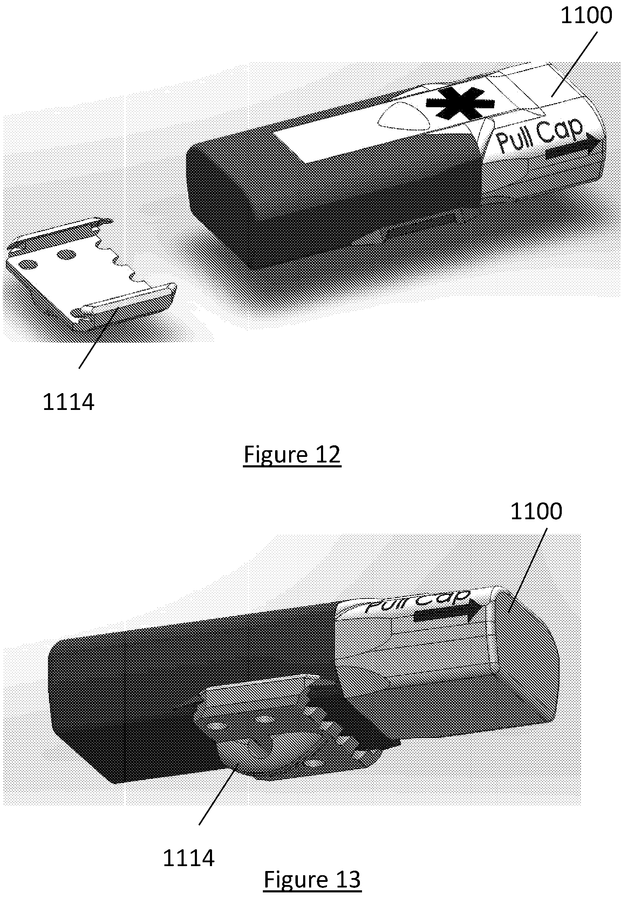

Figure 12 shows a top perspective view of the device 1100 with the slide plate 1114 removed therefrom. Figure 13 shows a bottom perspective view of the device 1100 with the slide plate 1114 attached thereto.

In some embodiments, a medication administration device as described herein (e.g., the device 100 or the device 1100) may be provided as a miniaturized wearable medication administration device. In some embodiments, a miniaturized wearable medication administration device having a quick release as described above can be of a size that is small enough to be conveniently attached to a user's person (e.g., clipped to a user's clothes) or coupled to another item (e.g., another accessory, a bag, a key chain, a backpack, a mobile phone case, a bracelet, a key ring, a bag, a lanyard, a magnet, a mobile phone, a pin, a belt, a carabiner, a backpack clip, a zipper pull, a wallet, a belt clip, etc.) for ease of carrying, transportation, and access, but is still sufficiently large so as to be easily held and manipulated by a user.

In some embodiments, a medication administration device as described herein (e.g., the device 100 or the device 1100) is configured for into a jewelry or wearable accessory that is adaptable to a wide variety of styles. In some embodiments, such a device includes one to two attachment sites through pins or clips that are compatible with the device and provide multiple functionalities. In some embodiments, the first site has a bail that effectively turns the device into a pendant or any accessory that is desired. In some embodiments, the second pin has a hinge knuckle onto which other parts (e.g., a cover, a second bail, a charm, etc.) can attach. In some embodiments, when a cover is used, the first pin has the additional functionality of being an attachment point for closure. In some embodiments, both attachment maximize unintentional interference with the device and support the device from opposing and/or pulling forces with minimal wear. In some embodiments, the attachment points work in harmony to transform the device into any wearable jewelry the user desires. In some embodiments, the attachment points also permit connection to other personal accessories or clothing such as cellphones or backpacks or belts.

In some embodiments, a medication administration device as described herein (e.g., the device 100 or the device 1100) has an activation trigger and a syringe located adjacent to one another at the same end of the device, and is activated by pressing the end of the device having the activation trigger against the skin of the patient at an appropriate location for administration of a therapeutic agent (e.g., the thigh). Consequently, in some embodiments, such a medication administration device can allow for administration of a therapeutic agent with a minimal amount of effort, and is suitable for use as an autoinjector (i.e., a device that is self-administered by a patient).

In some embodiments, an exemplary miniaturized wearable medication administration device (e.g., the device 100 described above) has a length (e.g., as measured between the outward-facing end of the removable cap 17 and the opposing outward-facing end of the coating 18) that is in a range of between 61.5 mm and 67.5 mm. In some embodiments, the length is between 62 mm and 67 mm. In some embodiments, the length is between 62.5 mm and 66.5 mm. In some embodiments, the length is between 63 mm and 66 mm. In some embodiments, the length is between 63.5 mm and 65.5 mm. In some embodiments, the length is between 64 mm and 65 mm. In some embodiments, the length is about 61.5 mm. In some embodiments, the length is 61.5 mm. In some embodiments, the length is about 62 mm. In some embodiments, the length is 62 mm. In some embodiments, the length is about 62.5 mm. In some embodiments, the length is 62.5 mm. In some embodiments, the length is about 63 mm. In some embodiments, the length is 63 mm. In some embodiments, the length is about 63.5 mm. In some embodiments, the length is 63.5 mm. In some embodiments, the length is about 64 mm. In some embodiments, the length is 64 mm. In some embodiments, the length is about 64.5 mm. In some embodiments, the length is 64.5 mm. In some embodiments, the length is about 65 mm. In some embodiments, the length is 65 mm. In some embodiments, the length is about 65.5 mm. In some embodiments, the length is 65.5 mm. In some embodiments, the length is about 66 mm. In some embodiments, the length is 66 mm. In some embodiments, the length is about 66.5 mm. In some embodiments, the length is 66.5 mm. In some embodiments, the length is about 67 mm. In some embodiments, the length is 67 mm. In some embodiments, the length is about 67.5 mm. In some embodiments, the length is 67.5 mm. In some embodiments, the length is less than 67.5 mm. In some embodiments, the length is between 10 mm and 67.5 mm.

In some embodiments, an exemplary miniaturized wearable medication administration device (e.g., the device 100 described above) has a width (e.g., as measured between opposing sides of the housing 1, as shown in Figure 5) that is in a range of between 23.6 mm and 27.6 mm. In some embodiments, the width is between 24.1 mm and 27.1 mm. In some embodiments, the width is between 24.6 mm and 26.6 mm. In some embodiments, the width is between 25.1 mm and 26.1 mm. In some embodiments, the width is about 23.1 mm. In some embodiments, the width is 23.1 mm. In some embodiments, the width is about 23.6 mm. In some embodiments, the width is 23.6 mm. In some embodiments, the width is about 24.1 mm. In some embodiments, the width is 24.1 mm. In some embodiments, the width is about 24.6 mm. In some embodiments, the width is 24.6 mm. In some embodiments, the width is about 25.1 mm. In some embodiments, the width is 25.1 mm. In some embodiments, the width is about 25.6 mm. In some embodiments, the width is 25.6 mm. In some embodiments, the width is about 26.1 mm. In some embodiments, the width is 26.1 mm. In some embodiments, the width is about 26.6 mm. In some embodiments, the width is 26.6 mm. In some embodiments, the width is about 27.1 mm. In some embodiments, the width is 27.1 mm. In some embodiments, the width is about 27.6 mm. In some embodiments, the width is 27.6 mm. In some embodiments, the width is about 24 mm. In some embodiments, the width is 24 mm. In some embodiments, the width is about 25 mm. In some embodiments, the width is 25 mm. In some embodiments, the width is about 26 mm. In some embodiments, the width is 26 mm. In some embodiments, the width is about 27 mm. In some embodiments, the width is 27 mm. In some embodiments, the width is less than 27 mm. In some embodiments, the width is between 5 mm and 27 mm.

In some embodiments, an exemplary miniaturized wearable medication administration device (e.g., the device 100 described above) has a height (e.g., as measured between the opposing top and bottom of the housing 1, as shown in Figure 5) that is in a range of between 12.6 mm and 16.6 mm. In some embodiments, the height is between 13.1 mm and 16.1 mm. In some embodiments, the height is between 13.6 mm and 15.6 mm. In some embodiments, the height is between 14.1 mm and 15.1 mm. In some embodiments, the height is about 12.6 mm. In some embodiments, the height is 12.6 mm. In some embodiments, the height is about 13.1 mm. In some embodiments, the height is 13.1 mm. In some embodiments, the height is about 13.6 mm. In some embodiments, the height is 13.6 mm. In some embodiments, the height is about 14.1 mm. In some embodiments, the height is 14.1 mm. In some embodiments, the height is about 14.6 mm. In some embodiments, the height is 14.6 mm. In some embodiments, the height is about 15.1 mm. In some embodiments, the height is 15.1 mm. In some embodiments, the height is about 15.6 mm. In some embodiments, the height is 15.6 mm. In some embodiments, the height is about 16.1 mm. In some embodiments, the height is 16.1 mm. In some embodiments, the height is about 16.6 mm. In some embodiments, the height is 16.6 mm. In some embodiments, the height is about 13 mm. In some embodiments, the height is 13 mm. In some embodiments, the height is about 14 mm. In some embodiments, the height is 14 mm. In some embodiments, the height is about 15 mm. In some embodiments, the height is 15 mm. In some embodiments, the height is about 16 mm. In some embodiments, the height is 16 mm. In some embodiments, the height is less than 16 mm. In some embodiments, the height is between 5 mm and 16 mm.

While a number of embodiments of the present invention have been described, it is understood that these embodiments are illustrative only, and not restrictive, and that many modifications may become apparent to those of ordinary skill in the art. For example, all dimensions discussed herein are provided as examples only, and are intended to be illustrative and not restrictive.

## Claims

1. A device, comprising:
- a housing (1) having a first end, a second end, a first cylinder (23) positioned within the housing (1), a second cylinder (25) positioned within the housing (1) and parallel to the first cylinder (23), and a channel connecting the first cylinder (23) to the second cylinder (25) proximate to the second end;
- a piston (4) slidably positioned within the first cylinder (23);
- a deployment spring (11) configured to force the piston (4) toward the second end of the housing (1);
- an activation trigger (2) positioned proximate to the first end of the housing (1), wherein the activation trigger (2) is configured to retain the piston (4) proximate the first end of the housing (1) prior to actuation of the activation trigger (2), and wherein the activation trigger (2) is configured to release the piston (4) and to allow the piston (4) to move along the first cylinder (23) upon actuation of the activation trigger (2);
- a penetrable septum (19) positioned at the first end of the housing (1) proximate to the activation trigger (2), wherein the penetrable septum (19) separates an interior of the housing (1) from an external environment surrounding the housing (1);
- a cartridge (6) having a first end and a second end, wherein the cartridge (6) contains at least one therapeutic agent, wherein the cartridge (6) is positioned within the second cylinder (25) such that the first end of the cartridge (6) faces the first end of the housing (1) and the second end of the cartridge (6) faces the second end of the housing (1), and wherein the cartridge (6) has a plunger (10) at the second end thereof;
- a needle hub (8) positioned adjacent to the first end of the cartridge (6) and within the second cylinder (25), wherein the cartridge (6) is slidably positioned within the needle hub (8);
- a dual-ended needle (7) positioned within the needle hub (8); and
- a fluid positioned within the first cylinder (23), the channel, and the second cylinder (25),
wherein the piston (4) is configured such that, when the activation trigger (2) is actuated, the activation trigger (2) releases the piston (4) and the deployment spring (11) causes the piston (4) to travel along the first cylinder (23) toward the second end of the housing (1), thereby driving at least some of the fluid through the channel into the second cylinder (25), thereby driving the cartridge (6), the needle hub (8), and the needle (7) toward the first end of the housing (1), and thereby causing the needle (7) to penetrate the septum (19) and subsequently to penetrate the cartridge (6); and
wherein the plunger (10) is configured such that continued travel of the fluid causes the plunger (10) to move within the cartridge (6) and force at least some of the at least one therapeutic agent to extrude through the needle (7).

2. The device of claim 1,
further comprising a retraction spring (12) positioned within the second cylinder (25), wherein the retraction spring (12) is configured to force the needle hub (8) toward the second end of the cylinder.

3. The device of claim 2,
further comprising a cartridge sealing ring (9)
wherein the piston (4) includes a piston head,
wherein the first cylinder (23) includes a relief bore (26) positioned proximate to the second end of the housing (1),
wherein the relief bore (26) is configured such that, when the piston (4) head is positioned between the relief bore (26) and the second end of the housing (1), the fluid is permitted to flow into a portion of the first cylinder (23) between the head of the piston (4) and the first end of the housing (1), thereby removing pressure from the cartridge sealing ring (9), and
wherein the retraction spring (12) is configured to force the needle hub (8) toward the second end of the housing (1) when pressure is removed from the cartridge sealing ring (9), thereby causing corresponding motion of the needle (7) toward the second end of the housing (1) and retraction of the needle (7) so as to be positioned entirely within the housing (1).

4. The device of claim 3,
wherein a position of the relief bore (26) is configured so as to control an amount of the at least one therapeutic agent to be extruded through the needle (7); and/or
wherein the deployment spring (11) and the retraction spring (12) are configured such that an elapsed time between an actuation of the activation trigger (2) and a subsequent retraction of the needle (7) is less than 2.0 seconds.

5. The device of one of claims 1 to 4,
wherein the fluid is one of a hydraulic fluid or a pneumatic fluid.

6. The device of one of claims 1 to 5,
wherein a length of the device is between 10 millimeters and 67.5 millimeters; and/or
wherein a height of the device is between 5 millimeters and 16 millimeters; and/or
wherein a width of the device is between 5 millimeters and 27 millimeters.

7. The device of one of claims 1 to 6,
further comprising a clip (14) configured to removably attach the device to at least one of a person or an item.

8. The device of claim 7,
wherein the clip includes at least one of a slide-on clip or a pinch clip (15).

9. The device of one of claims 1 to 8,
further comprising a cartridge sealing ring (9) positioned adjacent to the second end of the cartridge (6), wherein the fluid is configured to drive the cartridge sealing ring (9) toward the first end of the housing (1), thereby driving the cartridge (6) toward the first end of the housing (1).

10. The device of one of claims 1 to 9,
wherein the housing (1) is configured to reflect infrared radiation.

11. The device of one of claims 1 to 10,
wherein the housing (1) includes a semi-permeable barrier (28) overlaying the penetrable septum (19).

12. The device of claim 11,
wherein the semi-permeable barrier (28) comprises flash-spun high-density polyethylene.

13. The device of claim 11 or 12,
wherein the housing (1) is fluid-tight.

14. The device of one of claims 1 to 13,
wherein the at least one therapeutic agent is a plurality of therapeutic agents.

15. The device of one of claims 1 to 14,
further comprising at least one sensor.

## Patentansprüche

1. Ein System, umfassend:
- ein Gehäuse (1), das ein erstes Ende, ein zweites Ende, einen ersten Zylinder (23), der innerhalb des Gehäuses (1) positioniert ist, einen zweiten Zylinder (25), der innerhalb des Gehäuses (1) und parallel zum ersten Zylinder (23) positioniert ist, und einen Kanal, der den ersten Zylinder (23) mit dem zweiten Zylinder (25) nahe am zweiten Ende verbindet, aufweist;
- einen Kolben (4), der gleitend innerhalb des ersten Zylinders (23) positioniert ist;
- eine Auslösefeder (11), die so konfiguriert ist, dass sie den Kolben (4) in Richtung des zweiten Endes des Gehäuses (1) drückt;
- einen Aktivierungsauslöser (2), der nahe dem ersten Ende des Gehäuses (1) positioniert ist, wobei der Aktivierungsauslöser (2) so konfiguriert ist, dass er den Kolben (4) nahe dem ersten Ende des Gehäuses (1) hält, bevor der Aktivierungsauslöser (2) betätigt wird, und wobei der Aktivierungsauslöser (2) so konfiguriert ist, dass er den Kolben (4) freigibt und es dem Kolben (4) ermöglicht, sich beim Betätigen des Aktivierungsauslösers (2) entlang des ersten Zylinders (23) zu bewegen;
- ein durchdringbares Septum (19), das an dem ersten Ende des Gehäuses (1) in der Nähe des Aktivierungsauslösers (2) positioniert ist, wobei das durchdringbare Septum (19) ein Inneres des Gehäuses (1) von einer das Gehäuse (1) umgebenden Außenumgebung trennt;
- eine Kartusche (6) mit einem ersten Ende und einem zweiten Ende, wobei die Kartusche (6) mindestens einen therapeutischen Wirkstoff enthält, wobei die Kartusche (6) innerhalb des zweiten Zylinders (25) so positioniert ist, dass das erste Ende der Kartusche (6) dem ersten Ende des Gehäuses (1) zugewandt ist und das zweite Ende der Kartusche (6) dem zweiten Ende des Gehäuses (1) zugewandt ist, und wobei die Kartusche (6) an dessen zweitem Ende einen Stößel (10) aufweist;
- eine Nadelnabe (8), die neben dem ersten Ende der Kartusche (6) und innerhalb des zweiten Zylinders (25) positioniert ist, wobei die Kartusche (6) gleitend innerhalb der Nadelnabe (8) positioniert ist;
- eine doppelseitige Nadel (7), die innerhalb der Nadelnabe (8) positioniert ist; und
- ein Fluid, das innerhalb des ersten Zylinders (23), des Kanals und des zweiten Zylinders (25) positioniert ist,
wobei der Kolben (4) derart konfiguriert ist, dass, wenn der Aktivierungsauslöser (2) betätigt wird, der Aktivierungsauslöser (2) den Kolben (4) freigibt und die Auslösefeder (11) bewirkt, dass sich der Kolben (4) entlang des ersten Zylinders (23) in Richtung des zweiten Endes des Gehäuses (1) bewegt, wodurch mindestens ein Teil der Flüssigkeit durch den Kanal in den zweiten Zylinder (25) gedrückt wird, wodurch die Kartusche (6), die Nadelnabe (8) und die Nadel (7) in Richtung des ersten Endes des Gehäuses (1) gedrückt werden und dadurch die Nadel (7) dazu veranlasst wird, das Septum (19) zu durchdringen und anschließend die Kartusche (6) zu durchdringen; und
wobei der Stößel (10) so konfiguriert ist, dass eine kontinuierliche Bewegung der Flüssigkeit den Stößel (10) dazu bewirkt, sich innerhalb der Kartusche (6) zu bewegen und mindestens einen Teil des mindestens einen therapeutischen Wirkstoffs durch die Nadel (7) zu extrudieren.

2. Vorrichtung nach Anspruch 1,
ferner umfassend eine Rückzugfeder (12), die innerhalb des zweiten Zylinders (25) positioniert ist, wobei die Rückzugfeder (12) so konfiguriert ist, dass sie die Nadelnabe (8) in Richtung des zweiten Endes des Zylinders drückt.

3. Vorrichtung nach Anspruch 2,
ferner umfassend einen Patronendichtring (9) wobei der Kolben (4) einen Kolbenkopf beinhaltet,
wobei der erste Zylinder (23) eine Entlastungsbohrung (26) beinhaltet, die nahe dem zweiten Ende des Gehäuses (1) positioniert ist,
wobei die Entlastungsbohrung (26) so konfiguriert ist, dass, wenn der Kolbenkopf (4) zwischen der Entlastungsbohrung (26) und dem zweiten Ende des Gehäuses (1) positioniert ist, das Fluid in einen Teil des ersten Zylinders (23) zwischen dem Kopf des Kolbens (4) und dem ersten Ende des Gehäuses (1) fließen kann, wodurch der Druck vom Patronendichtring (9) abgebaut wird, und
wobei die Rückzugfeder (12) so konfiguriert ist, dass sie die Nadelnabe (8) in Richtung des zweiten Endes des Gehäuses (1) drückt, wenn der Druck vom Patronendichtring (9) abgebaut wird, wodurch eine entsprechende Bewegung der Nadel (7) in Richtung des zweiten Endes des Gehäuses (1) und ein Zurückziehen der Nadel (7) verursacht wird, sodass sie vollständig innerhalb des Gehäuses (1) positioniert ist.

4. Vorrichtung nach Anspruch 3,
wobei eine Position der Entlastungsbohrung (26) so eingerichtet ist, dass sie eine Menge des mindestens einen durch die Nadel (7) zu extrudierenden therapeutischen Wirkstoffs steuert; und/oder
wobei die Auslösefeder (11) und die Rückzugfeder (12) so konfiguriert sind, dass eine zwischen einer Betätigung des Aktivierungsauslösers (2) und einem anschließenden Zurückziehen der Nadel (7) vergangene Zeit weniger als 2,0 Sekunden beträgt.

5. Vorrichtung nach einem der Ansprüche 1 bis 4,
wobei die Flüssigkeit eine Hydraulikflüssigkeit oder eine pneumatische Flüssigkeit ist.

6. Vorrichtung nach einem der Ansprüche 1 bis 5,
wobei eine Länge der Vorrichtung zwischen 10 Millimetern und 67,5 Millimetern beträgt; und/oder
wobei eine Höhe der Vorrichtung zwischen 5 Millimetern und 16 Millimetern beträgt; und/oder
wobei eine Breite der Vorrichtung zwischen 5 Millimetern und 27 Millimetern beträgt.

7. Vorrichtung nach einem der Ansprüche 1 bis 6,
ferner umfassend einen Clip (14), der dazu konfiguriert ist, die Vorrichtung abnehmbar an mindestens einer Person oder einem Gegenstand zu befestigen.

8. Vorrichtung nach Anspruch 7,
wobei der Clip mindestens einen von einem Aufschiebeclip oder einem Quetschclip (15) beinhaltet.

9. Vorrichtung nach einem der Ansprüche 1 bis 8,
ferner umfassend einen Patronendichtring (9), der neben dem zweiten Ende der Patrone (6) positioniert ist, wobei das Fluid so eingerichtet ist, dass es den Patronendichtring (9) in Richtung des ersten Endes des Gehäuses (1) antreibt, wodurch die Patrone (6) in Richtung des ersten Endes des Gehäuses (1) angetrieben wird.

10. Vorrichtung nach einem der Ansprüche 1 bis 9,
wobei das Gehäuse (1) so konfiguriert ist, dass es Infrarotstrahlung reflektiert.

11. Vorrichtung nach einem der Ansprüche 1 bis 10,
wobei das Gehäuse (1) eine halbdurchlässige Barriere (28) beinhaltet, die das durchlässige Septum (19) überlagert.

12. Vorrichtung nach Anspruch 11,
wobei die halbdurchlässige Barriere (28) fließgesponnenes Polyethylen hoher Dichte umfasst.

13. Vorrichtung nach Anspruch 11 oder 12,
wobei das Gehäuse (1) flüssigkeitsdicht ist.

14. Vorrichtung nach einem der Ansprüche 1 bis 13,
wobei der mindestens eine therapeutische Wirkstoff eine Vielzahl von therapeutischen Wirkstoffen ist.

15. Vorrichtung nach einem der Ansprüche 1 bis 14,
ferner umfassend mindestens einen Sensor.

## Revendications

1. Dispositif, comprenant :
- un boîtier (1) présentant une première extrémité, une deuxième extrémité, un premier cylindre (23) positionné à l'intérieur du boîtier (1), un deuxième cylindre (25) positionné à l'intérieur du boîtier (1) et parallèle au premier cylindre (23), et un canal reliant le premier cylindre (23) au deuxième cylindre (25) à proximité de la deuxième extrémité ;
- un piston (4) positionné de manière coulissante à l'intérieur du premier cylindre (23) ;
- un ressort de déploiement (11) configuré pour forcer le piston (4) vers la deuxième extrémité du boîtier (1) ;
- un déclencheur d'activation (2) positionné à proximité de la première extrémité du boîtier (1), dans lequel le déclencheur d'activation (2) est configuré pour retenir le piston (4) à proximité de la première extrémité du boîtier (1) avant l'actionnement du déclencheur d'activation (2), et dans lequel le déclencheur d'activation (2) est configuré pour libérer le piston (4) et pour permettre au piston (4) de se déplacer le long du premier cylindre (23) lors de l'actionnement du déclencheur d'activation (2) ;
- un septum (19) pénétrable positionné à la première extrémité du boîtier (1) à proximité du déclencheur d'activation (2), dans lequel le septum pénétrable (19) sépare un intérieur du boîtier (1) d'un environnement externe entourant le boîtier (1) ;
- une cartouche (6) présentant une première extrémité et une deuxième extrémité, dans lequel la cartouche (6) contient au moins un agent thérapeutique, dans lequel la cartouche (6) est positionnée à l'intérieur du deuxième cylindre (25) de sorte que la première extrémité de la cartouche (6) soit orientée vers la première extrémité du boîtier (1) et que la deuxième extrémité de la cartouche (6) soit orientée vers la deuxième extrémité du boîtier (1), et dans lequel la cartouche (6) présente un poussoir (10) à la deuxième extrémité de celle-ci ;
- une embase d'aiguille (8) positionnée de manière adjacente à la première extrémité de la cartouche (6) et à l'intérieur du deuxième cylindre (25), dans lequel la cartouche (6) est positionnée de manière coulissante à l'intérieur de l'embase d'aiguille (8) ;
- une aiguille (7) à double extrémité positionnée à l'intérieur de l'embase d'aiguille (8) ; et
- un fluide positionné à l'intérieur du premier cylindre (23), du canal, et du deuxième cylindre (25),
dans lequel le piston (4) est configuré de sorte que, lorsque le déclencheur d'activation (2) est actionné, le déclencheur d'activation (2) libère le piston (4) et le ressort de déploiement (11) amène le piston (4) à se déplacer le long du premier cylindre (23) vers la deuxième extrémité du boîtier (1), entraînant ainsi au moins une certaine partie du fluide à travers le canal dans le deuxième cylindre (25), entraînant ainsi la cartouche (6), l'embase d'aiguille (8) et l'aiguille (7) vers la première extrémité du boîtier (1), et amenant ainsi l'aiguille (7) à pénétrer le septum (19) et ensuite à pénétrer la cartouche (6) ; et
dans lequel le poussoir (10) est configuré de sorte que le déplacement continu du fluide amène le poussoir (10) à se déplacer à l'intérieur de la cartouche (6) et force au moins une certaine partie de l'au moins un agent thérapeutique à sortir par l'aiguille (7).

2. Dispositif selon la revendication 1,
comprenant en outre un ressort de rétraction (12) positionné à l'intérieur du deuxième cylindre (25), dans lequel le ressort de rétraction (12) est configuré pour forcer l'embase d'aiguille (8) vers la deuxième extrémité du cylindre.

3. Dispositif selon la revendication 2,
comprenant en outre une bague d'étanchéité de cartouche (9)
dans lequel le piston (4) comporte une tête de piston, dans lequel le premier cylindre (23) comporte un alésage de décharge (26) positionné à proximité de la deuxième extrémité du boîtier (1),
dans lequel l'alésage de décharge (26) est configuré de sorte que, lorsque la tête du piston (4) est positionnée entre l'alésage de décharge (26) et la deuxième extrémité du boîtier (1), le fluide est autorisé à s'écouler dans une partie du premier cylindre (23) entre la tête du piston (4) et la première extrémité du boîtier (1), éliminant ainsi la pression de la bague d'étanchéité de cartouche (9), et
dans lequel le ressort de rétraction (12) est configuré pour forcer l'embase d'aiguille (8) vers la deuxième extrémité du boîtier (1) lorsque la pression est retirée de la bague d'étanchéité de cartouche (9), provoquant ainsi un mouvement correspondant de l'aiguille (7) vers la deuxième extrémité du boîtier (1) et une rétraction de l'aiguille (7) de manière à ce qu'elle soit positionnée entièrement à l'intérieur du boîtier (1).

4. Dispositif selon la revendication 3,
dans lequel une position de l'alésage de décharge (26) est configurée de manière à contrôler une quantité de l'au moins un agent thérapeutique à faire sortir par l'aiguille (7) ; et/ou
dans lequel le ressort de déploiement (11) et le ressort de rétraction (12) sont configurés de sorte qu'un temps écoulé entre un actionnement du déclencheur d'activation (2) et une rétraction ultérieure de l'aiguille (7) est inférieur à 2,0 secondes.

5. Dispositif selon l'une quelconque des revendications 1 à 4,
dans lequel le fluide est l'un parmi un fluide hydraulique ou un fluide pneumatique.

6. Dispositif selon l'une quelconque des revendications 1 à 5,
dans lequel une longueur du dispositif est comprise entre 10 millimètres et 67,5 millimètres ; et/ou
dans lequel une hauteur du dispositif est comprise entre 5 millimètres et 16 millimètres ; et/ou
dans lequel une largeur du dispositif est comprise entre 5 millimètres et 27 millimètres.

7. Dispositif selon l'une quelconque des revendications 1 à 6,
comprenant en outre un clip (14) configuré pour fixer de manière amovible le dispositif à au moins l'un parmi une personne ou un article.

8. Dispositif selon la revendication 7,
dans lequel le clip comporte au moins l'un parmi un clip coulissant ou un clip de pincement (15).

9. Dispositif selon l'une quelconque des revendications 1 à 8,
comprenant en outre une bague d'étanchéité de cartouche (9) positionnée de manière adjacente à la deuxième extrémité de la cartouche (6), dans lequel le fluide est configuré pour entraîner la bague d'étanchéité de cartouche (9) vers la première extrémité du boîtier (1), entraînant ainsi la cartouche (6) vers la première extrémité du boîtier (1).

10. Dispositif selon l'une quelconque des revendications 1 à 9,
dans lequel le boîtier (1) est configuré pour réfléchir un rayonnement infrarouge.

11. Dispositif selon l'une quelconque des revendications 1 à 10,
dans lequel le boîtier (1) comporte une barrière semi-perméable (28) recouvrant le septum (19) pénétrable.

12. Dispositif selon la revendication 11,
dans lequel la barrière semi-perméable (28) comprend du polyéthylène haute densité obtenu par filage éclair.

13. Dispositif selon la revendication 11 ou 12,
dans lequel le boîtier (1) est étanche aux fluides.

14. Dispositif selon l'une quelconque des revendications 1 à 13,
dans lequel l'au moins un agent thérapeutique est une pluralité d'agents thérapeutiques.

15. Dispositif selon l'une quelconque des revendications 1 à 14,
comprenant en outre au moins un capteur.
